# EUROPÄISCHE PATENTSCHRIFT

(11) **EP 0 770 052 B1**
(45) Veröffentlichungstag und Bekanntmachung des Hinweises auf die Patenterteilung: **31.03.1999**
(21) Anmeldenummer: 95925805.4
(22) Anmeldetag: 03.07.1995
(51) Int. Cl.: C07C 51/14, C07C 53/122

(54) **VERFAHREN ZUR CARBONYLIERUNG VON OLEFINEN**
PROCESS FOR CARBONYLATING OLEFINS
PROCEDE DE CARBONYLATION DES OLEFINES

(30) Priorität: 13.07.1994 DE 4424710
(43) Veröffentlichungstag der Anmeldung: 02.05.1997
(73) Patentinhaber: BASF AKTIENGESELLSCHAFT, 67056 Ludwigshafen (DE)
(72) Erfinder: LIPPERT, Ferdinand, D-67098 Bad Dürkheim (DE); HÖHN, Arthur, D-67281 Kirchheim (DE); SCHÄFER, Martin, D-67063 Ludwigshafen (DE); HUPFER, Leopold, D-67159 Friedelsheim (DE)
(86) Internationale Anmeldenummer: EP9502560
(87) Internationale Veröffentlichungsnummer: WO9602487

(56) Entgegenhaltungen:
- DE-A- 2 057 521
- DE-C- 863 194
- DE-C- 868 150

## Beschreibung

Die vorliegende Erfindung betrifft ein verbessertes Verfahren zur Carbonylierung von Olefinen mit Kohlenmonoxid in Gegenwart von Wasser, eines Alkohols oder einer Carbonsäure sowie eines halogenfreien Katalysators.

Aus der DE-C 863 194 und der DE-C 868 150 ist die Carbonylierung von Olefinen nach dem Reppe-Verfahren in Gegenwart metallcarbonylbildender Metalle oder von deren halogenfreien Verbindungen bekannt.

Auch Weissermel et al. beschreiben in Industrielle Organische Chemie, 1978, 2. Auflage, Verlag Chemie, S. 132 die Carbonylierung von Olefinen nach dem Reppe-Verfahren, beispielsweise die Herstellung von Propionsäure aus Ethylen, Kohlenmonoxid und Wasser in Gegenwart von Katalysatoren. Als Katalysator wird Nickelpropionat eingesetzt, das sich unter den Reaktionsbedingungen zu Nickeltetracarbonyl umsetzt. Ein hoher Umsatz des Kohlenmonoxids wird bei den vorgenannten Reppe-Verfahren nur bei hohen Drücken (200 bis 240 bar) und Temperaturen (270 bis 320°C) erzielt. Diese Reaktionsbedingungen erfordern einen hohen technischen Aufwand beim Bau geeigneter Reaktoren sowie - bedingt durch die Korrosivität des Produktes unter den Reaktionsbedingungen - besondere und teure Werkstoffe.

Die GB-A 1 063 617 lehrt die Carbonylierung von Olefinen mit Nickel- und Cobaltkatalysatoren in Gegenwart von Borsäure. Auch hierzu sind hohe Drücke und Temperaturen erforderlich.

Carbonylierungen von Olefinen können mit Edelmetallkatalysatoren bei Drücken von ca. 100 bar durchgeführt werden. So lehrt die EP-A 495 547 Katalysatoren, die aus einer Palladiumquelle und zweizähnigen Phosphinliganden bestehen. Solche Katalysatoren werden aber häufig nach kurzer Reaktionsdauer durch Abscheidung metallischen Palladiums desaktiviert; insbesondere sind die eingesetzten Phosphinliganden unter den gewünschten Reaktionsbedingungen nicht thermostabil.

Die EP-A 329 252 betrifft ein Katalysatorsystem zur Carbonylierung von Olefinen, das eine Rutheniumkomponente sowie eine Sauerstoffverbindung von Phosphor, Antimon, Arsen, Molybdän oder Wolfram umfaßt. Solche Katalysatoren sind jedoch nur schwach aktiv, so daß sie für einen großtechnischen Einsatz nur wenig geeignet sind.

Es stellte sich die Aufgabe, ein Verfahren zur Carbonylierung von Olefinen bereitzustellen, das die oben angegebenen Nachteile vermeidet.

Demgemäß wurde ein Verfahren zur Carbonylierung von Olefinen mit Kohlenmonoxid in Gegenwart von Wasser, eines Alkohols oder einer Carbonsäure sowie eines halogenfreien Katalysators gefunden, das dadurch gekennzeichnet ist, daß man als Katalysator Nickel und Ruthenium oder Nickel und Platin oder eine Verbindung dieser Metalle verwendet.

Die folgende Reaktionsgleichung verdeutlicht das erfindungsgemäße Verfahren am Beispiel der Umsetzung von Ethylen zu Propionsäure:

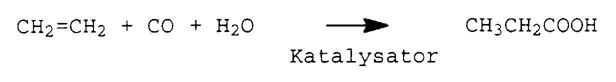

Als Ausgangsstoffe für das erfindungsgemäße Verfahren kommen aliphatische und cycloaliphatische Alkene mit vorzugsweise 2 bis 50, besonders bevorzugt 2 bis 7 Kohlenstoffatomen in Betracht. Beispielhaft sind Ethylen, Propylen, iso-Buten, 1-Buten, 2-Buten und die Isomeren des Pentens und Hexens sowie Cyclohexen zu nennen, von denen Ethylen bevorzugt ist.

Diese Olefine werden zur Herstellung von Carbonsäuren mit Wasser, zur Herstellung von Carbonsäureestern mit Alkoholen und zur Herstellung von Säureanhydriden mit Carbonsäuren umgesetzt.

Die Alkohole umfassen aliphatische und cycloaliphatische Verbindungen mit bevorzugt 1 bis 20 Kohlenstoffatomen, besonders bevorzugt mit 1 bis 6 Kohlenstoffatomen, wie Methanol, Ethanol, n-Propanol, iso-Propanol, iso-Butanol, tert.-Butanol, Stearylalkohol, Diole wie Ethylenglykol, 1,2-Propandiol und 1,6-Hexandiol sowie Cyclohexanol. Werden Diole umgesetzt, können je nach den gewählten stöchiometrischen Verhältnissen Mono- und Diester erhalten werden, wobei zur Herstellung von Diestern das Diol und das Olefin im molaren Verhältnis von etwa 1:2 und zur Herstellung der Monoester das Diol im Überschuß eingesetzt werden.

Erfolgt die Carbonylierung des Olefins in Gegenwart von wasserfreien Carbonsäuren, werden Carbonsäureanhydride gebildet. Es können je nach Wahl der Carbonsäure bevorzugt symmetrische, aber auch asymmetrische Carbonsäureanhydride hergestellt werden.

Die genannten Ausgangsverbindungen werden mit Kohlenmonoxid umgesetzt, wobei dieses in reiner Form oder mit inerten Gasen wie Stickstoff oder Argon verdünnt eingesetzt werden kann.

Die molaren Verhältnisse der Ausgangsverbindungen Olefin und Wasser, Alkohol bzw. Carbonsäure können in weiten Grenzen variieren, jedoch wird in der Regel mindestens eine äquimolare Menge an Wasser, Alkohol bzw. Carbonsäure, bezogen auf das Olefin, eingesetzt. Insbesondere bei der Herstellung von Carbonsäuren kann ein großer Wasserüberschuß gewählt werden, z.B. 2 bis 10 mol Wasser pro Mol Olefin.

Auch das Verhältnis von Olefin zu Kohlenmonoxid kann stark variiert werden, z.B. zwischen 5:1 und 1:5 mol Olefin pro mol Kohlenmonoxid.

Als Katalysatoren werden im erfindungsgemäßen Verfahren Kombinationen von Nickel und Ruthenium oder Nickel und Platin oder deren Verbindungen eingesetzt. Damit sich die aktiven Verbindungen des Nickels bilden können, werden dem Reaktionsgemisch zweckmäßigerweise darin lösliche Nickelverbindungen zugesetzt, beispielsweise Acetat, Propionat, Acetylacetonat, Hydroxid und Carbonat oder Mischungen dieser Verbindungen. Besonders bevorzugt ist es, die Nickelkomponente zur Herstellung von Carbonsäuren in Form von Carbonsäuresalzen der bei der Reaktion gebildeten Carbonsäuren einzutragen. Es ist aber auch möglich, Nickelmetall in das Reaktionsgemisch einzutragen. Auch Nickel auf inerten Trägern wie Aktivkohle kann verwendet werden.

Die Metalle können beispielsweise als Salze, wie sie oben für Nickel angegeben sind, in die Reaktionslösung eingebracht werden, d.h. als Acetate, Propionate, Acetylacetonate, Hydroxide oder Carbonate. Weiterhin kommen Carbonylverbindungen in Betracht, insbesondere Trirutheniumdodecacarbonyl und Carbonylverbindungen, die weitere Liganden tragen oder durch Donorliganden wie Phosphine, Arsine und Stickstoffbasen sowie Olefine stabilisierte Metallverbindungen. Platin kann auch als Dibenzalacetonplatin eingesetzt werden. Ruthenium wird vorzugsweise als Acetylacetonat eingesetzt. Die Metalle liegen in der Reaktionsmischung je nach den Löslichkeitsverhältnissen gelöst oder suspendiert vor. Die Metalle oder Metallverbindungen können auch auf organischen oder anorganischen inerten Trägern wie Aktivkohle, Graphit, Aluminiumoxid, Zirkoniumdioxid und Siliciumoxid verwendet werden, z.B. Pt auf Aktivkohle und Pd auf Aktivkohle.

Das Molverhältnis von Nickel zum weiteren Metall liegt im allgemeinen bei 1:10 bis 10000:1, bevorzugt bei 1:1 bis 500:1. Der Gehalt der Reaktionslösung an den katalytisch aktiven Metallen beträgt im allgemeinen 0,01 bis 5 Gew.-%, berechnet als Metall.

Die Reaktion kann ohne oder in einem Lösungsmittel durchgeführt werden. Dazu kommen Lösungsmittel wie Aceton, Ether wie Dioxan, Dimethoxyethan, Tetraethylenglykoldimethylether, aprotische polare Lösungsmittel wie N-Methylpyrrolidon, Dimethylpropylenharnstoff und aromatische Kohlenwasserstoffe wie Toluol und Xylol in Betracht. Carbonsäuren können in Wasser als Lösungsmittel hergestellt werden. Bei der Herstellung einer Carbonsäure ist es bevorzugt, die Reaktion in dieser Carbonsäure, welche 10 bis 90 Gew.-% Wasser enthält, als Lösungsmittel durchzuführen. Nach dem erfindungsgemäßen Verfahren wird Propionsäure in 10 bis 90 Gew.-% Wasser enthaltender Propionsäure als Lösungsmittel hergestellt. Entsprechend können die Ester bevorzugt im jeweiligen Alkohol als Lösungsmittel hergestellt werden. Carbonsäureanhydride werden bevorzugt in wasserfreien Carbonsäuren hergestellt, so läßt sich Ethylen in Gegenwart wasserfreier Propionsäure zu Propionsäureanhydrid umsetzen.

Die Reaktion wird in der Regel bei 100 bis 300°C, vorzugsweise 170 bis 250°C und Drücken von 30 bis 150 bar, vorzugsweise 75 bis 120 bar durchgeführt.

Die Ausgangsverbindungen Olefin, Wasser, Alkohol bzw. Carbonsäure und der Katalysator können vor der Umsetzung, gegebenenfalls in einem Lösungsmittel, in einem Reaktor vermischt werden. Sie können dann auf die Reaktionstemperatur erhitzt werden. Der Reaktionsdruck kann durch Aufpressen von Kohlenmonoxid oder beim Einsatz kurzkettiger Olefine durch Aufpressen eines Gemisches aus diesem Olefin und Kohlenmonoxid eingestellt werden.

In der Regel ist die Umsetzung nach 0,5 bis 3 h beendet. Sie kann kontinuierlich oder diskontinuierlich in Reaktoren wie Kesseln, Blasensäulen oder Rohrreaktoren ausgeführt werden.

Zur Isolierung der Verfahrensprodukte wird der Reaktionsaustrag in einer bevorzugten Ausführungsform entspannt. Dann wird Nickelcarbonyl durch Durchleiten von Inertgas wie Stickstoff aus der Flüssigkeit ausgetragen. Nickelcarbonyl kann vom Inertgas abgetrennt und zu einer Nickelverbindung aufgearbeitet werden, welche wieder in die Reaktion zurückgeführt werden kann. Die Flüssigphase des Reaktionsaustrags, die neben dem Verfahrensprodukt lösliche oder unlösliche Katalysatorbestandteile enthält, wird gegebenenfalls filtriert und dann destillativ aufgearbeitet, wobei das Verfahrensprodukt, gegebenenfalls nach einer anschließenden Feindestillation, isoliert wird. Der katalysatorhaltige Destillationssumpf wird in die Reaktion zurückgeführt. Ebenso können gegebenenfalls vor der Destillation abfiltrierte Katalysatorbestandteile nach entsprechender Aufarbeitung zurückgeführt werden.

Das erfindungsgemäße Verfahren erlaubt die Herstellung der Verfahrensprodukte in hoher Raum-Zeit-Ausbeute mit hoher Selektivität unter moderaten Reaktionsbedingungen.

Die Verfahrensprodukte dienen als Zwischenprodukte zur Herstellung einer Vielzahl von Verbindungen, z.B. von Carboxylatgruppen tragenden Monomeren, weiterhin als Konservierungsmittel (Propionsäure) und als Lösungsmittel, z.B. für Lacke.

### Beispiele

### Diskontinuierliche Versuche zur Herstellung von Propionsäure

In einem Autoklaven wurden Katalysator (Ni als Nickelpropionat, Ru als Rutheniumacetylacetonat) und ein Gemisch aus 60 g Propionsäure und 40 g Wasser vorgelegt. Bei 200°C wurde mit einem Gemisch aus 50 Vol.-% Ethylen und 50 Vol.-% CO ein Druck von 100 bar eingestellt und durch Nachdrücken gehalten. Nach 2 h wurde entspannt und der Reaktionsaustrag titrimetrisch und gaschromatographisch analysiert. Die Ergebnisse sind in der folgenden Tabelle zusammengefaßt:

| Beispiel | Katalysator [mmol] | | RZA [g PS/(l·.h)] | Selektivität [%] | | |
|---|---|---|---|---|---|---|
| | Ni | Ru | | PS | PA | DEK |
| 1 | 16,99 | 0,075 | 175 | 86 | 1,1 | 1,6 |
| 2 (Vergleich) | 17,07 | - | 9 | 88 | 0 | 3,6 |
| 3 (Vergleich) | - | 17,07 | 17 | 28 | 0 | 4,2 |
| RZA = Raum-Zeit-Ausbeute | | | | | | |
| PS = Propionsäure | | | | | | |
| PA = Propionaldehyd, Nebenprodukt | | | | | | |
| DEK = Diethylketon, Nebenprodukt | | | | | | |

Im erfindungsgemäßen Beispiel 1 mit Ni und Ru wurden bei gleicher Molzahl an Katalysatormetall deutlich höhere Raum-Zeit-Ausbeuten und Selektivitäten erzielt als in den Versuchen mit jeweils nur einer Metallverbindung als Katalysator.

### Kontinuierliche Versuche zur Herstellung von Propionsäure

Eine Mischung aus Propionsäure, Wasser und Nickelpropionat sowie Rutheniumacetylacetonat wurde kontinuierlich bei 200°C und 100 bar mit einem Gemisch aus 50 Vol.-% Ethylen und 50 Vol.-% CO umgesetzt. Bei einer mittleren Verweilzeit VWZ zwischen 0,5 und 1 h wurde kontinuierlich Reaktionsgemisch entnommen und analysiert. Die folgende Tabelle gibt die Ergebnisse der Versuche an:

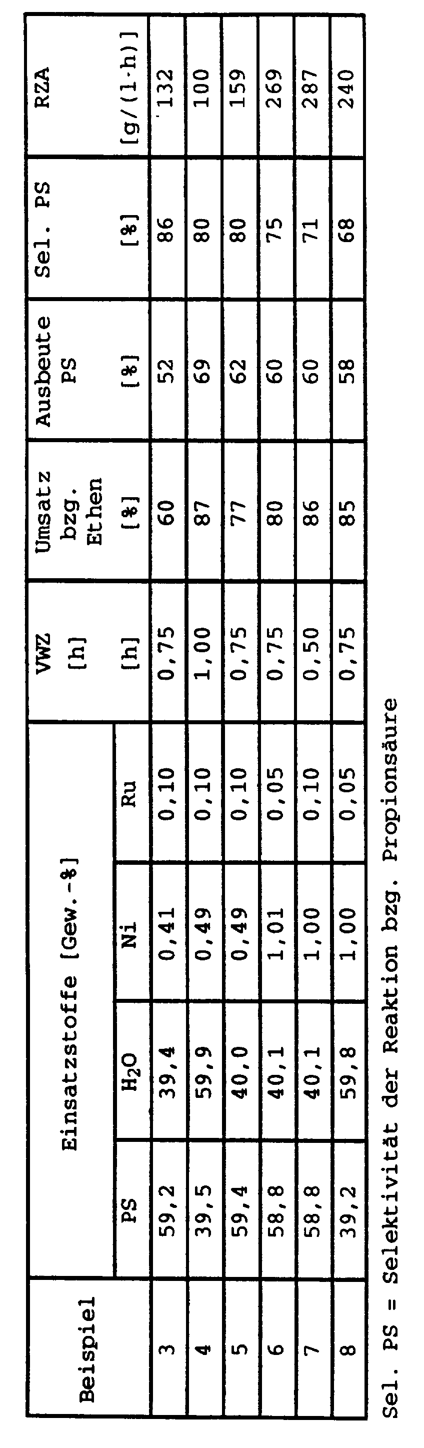

Das erfindungsgemäß Verfahren erlaubt die Herstellung von Propionsäure in hoher Ausbeute bei guter Raum-Zeit-Ausbeute. Druck und Temperatur sind dabei deutlich niedriger als bei der alleinigen Verwendung von Nickel als Katalysator.

### Beispiele

### Diskontinuierliche Versuche zur Herstellung von Propionsäure

In einem Autoklaven wurde der Nickelkatalysator (als Nickelpropionat), der Kokatalysator (siehe Tabelle) und ein Gemisch aus 60 g Propionsäure und 40 g Wasser vorgelegt. Bei 200°C wurde mit einem Gemisch aus 50 Vol.-% Ethen und 50 Vol.-% CO ein Druck von 100 bar eingestellt und durch Nachdrücken gehalten. Nach 2 h wurde entspannt und der Reaktionsaustrag titrimetrisch und gaschromatographisch analysiert. Die Ergebnisse sind in der folgenden Tabelle zusammengefaßt:

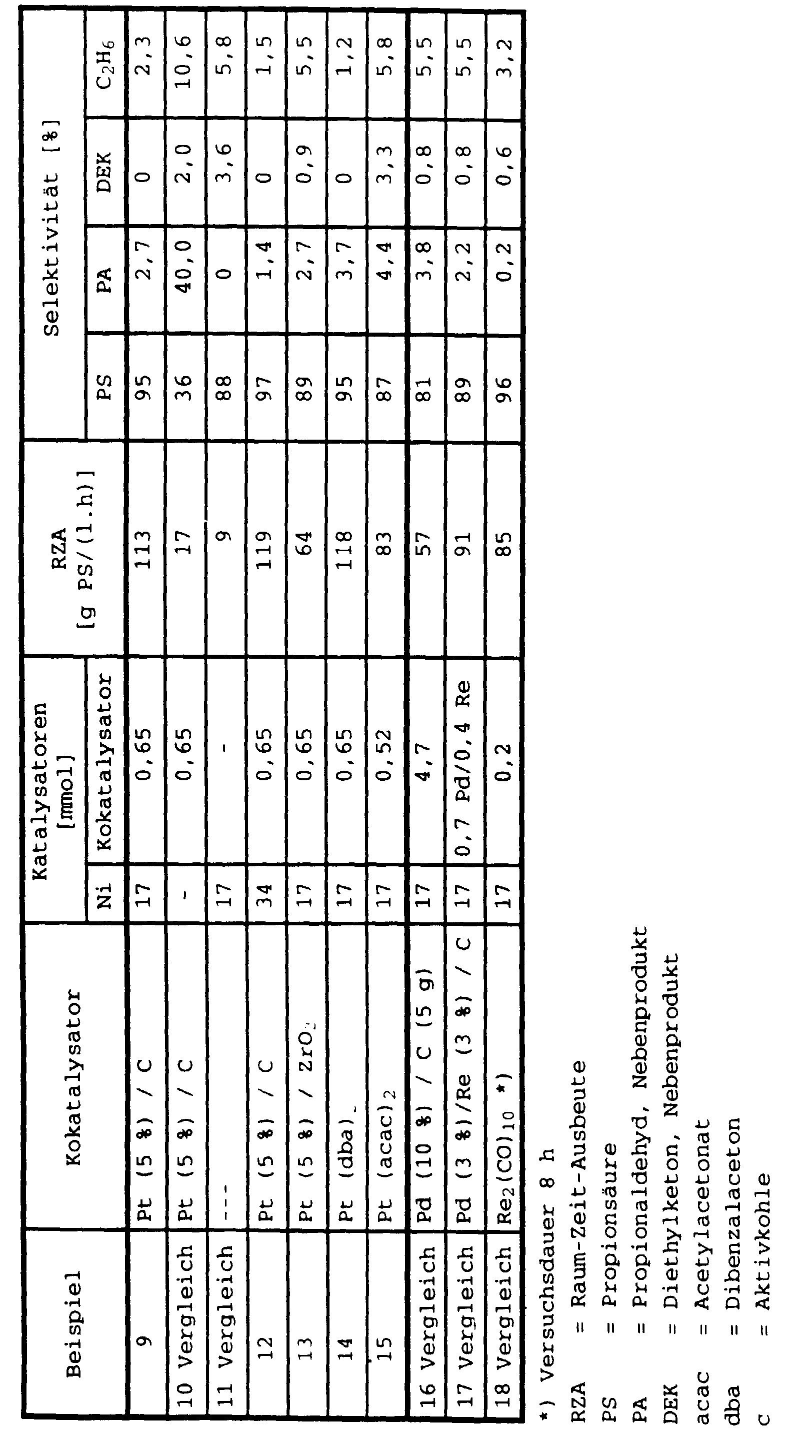

## Patentansprüche

1. Verfahren zur Carbonylierung von Olefinen mit Kohlenmonoxid in Gegenwart von Wasser, eines Alkohols oder einer Carbonsäure sowie eines halogenfreien Katalysators, dadurch gekennzeichnet, daß man als Katalysator Nickel und Ruthenium oder Nickel und Platin oder jeweils deren Verbindungen verwendet.

2. Verfahren nach Anspruch 1, dadurch gekennzeichnet, daß man die Carbonylierung bei einer Temperatur von 170 bis 250°C und bei einem Druck von 30 bis 150 bar vornimmt.

3. Verfahren nach den Ansprüchen 1 oder 2, dadurch gekennzeichnet, daß man Ethylen in Gegenwart von Wasser zu Propionsäure carbonyliert.

4. Verfahren nach Anspruch 3, dadurch gekennzeichnet, daß man die Herstellung von Propionsäure in 10 bis 90 Gew.-% Wasser enthaltender Propionsäure als Lösungsmittel vornimmt.

5. Verfahren nach den Ansprüchen 1 oder 2, dadurch gekennzeichnet, daß man Ethylen in Gegenwart wasserfreier Propionsäure zu Propionsäureanhydrid umsetzt.

## Claims

1. A process for carbonylating olefins with carbon monoxide in the presence of water, an alcohol or a carboxylic acid and a halogen-free catalyst, which comprises using nickel and ruthenium or nickel and platinum or in each case compounds thereof as catalyst.

2. A process as claimed in claim 1 wherein the carbonylation is carried out at a temperature from 170 to 250°C and at a pressure from 30 to 150 bar.

3. A process as claimed in claim 1 or 2 wherein ethylene is carbonylated in the presence of water to form propionic acid.

4. A process as claimed in claim 3 wherein the synthesis of propionic acid is carried out in a solvent comprising propionic acid having a water content from 10 to 90% by weight.

5. A process as claimed in claim 1 or 2 wherein ethylene is reacted in the presence of water-free propionic acid to form propionic anhydride.

## Revendications

1. Procédé de carbonylation d'oléfines avec du monoxyde de carbone en présence d'eau, d'un alcool ou d'un acide carboxylique, ainsi que d'un catalyseur non-halogéné, caractérisé en ce qu'on utilise en tant que catalyseur du nickel et du ruthénium ou du nickel et du platine ou leurs composés.

2. Procédé selon la revendication 1, caractérisé en ce qu'on met en oeuvre la carbonylation à une température de 170 à 250°C et sous une pression de 30 à 150 bar.

3. Procédé selon les revendications 1 ou 2, caractérisé en ce qu'on carbonyle de l'éthylène en acide propionique en présence d'eau.

4. Procédé selon la revendication 3, caractérisé en ce qu'on met en oeuvre la préparation de l'acide propionique avec, en tant que solvant, de l'acide propionique contenant de 10 à 90 % en poids d'eau.

5. Procédé selon les revendications 1 ou 2, caractérisé en ce qu'on fait réagir de l'éthylène en présence d'acide propionique anhydre pour obtenir de l'anhydride propionique.
